# EUROPEAN PATENT SPECIFICATION

(11) **EP 1 321 761 B1**
(45) Date of publication and mention of the grant of the patent: **23.08.2006**
(21) Application number: 02447264.9
(22) Date of filing: 20.12.2002
(51) Int. Cl.: G01N 21/75

(54) **Method of detecting an analyte**
Verfahren zum Nachweis eines Analyten
Procédé de détection d'analytes

(30) Priority: 21.12.2001 US 345169 P
(43) Date of publication of application: 25.06.2003
(73) Proprietor: Interuniversitair Micro-Elektronica Centrum, 3001 Heverlee (BE)
(72) Inventor: Frederix, Filip, 3511 Hasselt (BE); Borghs, Gustaaf, 3010 Leuven (BE); Friedt, Jean-Michel, 92200 Neuilly sur Seine (FR)
(74) Representative: Van Malderen, Joëlle

(56) References cited:
- EP-A- 0 469 377
- WO-A-01/44813
- US-B1- 6 268 125

## Description

### Field of the invention

The present invention is related to different types of biosensors which are known with their specific advantages and disadvantages. Electrochemical biosensors, Surface Acoustic Wave sensors and Surface Plasmon Resonance biosensors are examples of biosensors which do not require labelling techniques.

### State of the art

US 5,641,640 discloses a method for assaying an analyte in a fluid sample using surface plasmon resonance. The presence of an analyte is determined by the change in refractive index when the analyte interacts with a refractive index enhancing species. Surface plasmon resonance measurements have some major disadvantages such as :
1) most systems are rather expensive. The system requires a quartz prisma and a radiation source that is capable of generating polarized light.
2) SPR response depends on the volume and refractive index of the bound analyte. For very small molecules, this results in very small changes of refractive index.
The receptors should be immobilized on the surface.

US 6,330,464 discloses an optical based sensing device for detecting the presence of an amount of analyte using both indicator and reference channels. The sensor has a sensor body with a source of radiation therein. Radiation emitted by the source interacts with molecules, resulting in a change of at least one optical characteristic of those molecules.

WO 01/44813 A discloses a method of assaying an analyte in a sample by contacting the sample with a sensor device, the sensor device comprising a polymer film coated with a metal and having an analyte-specific receptor layer printed in a pattern on the metal coating, transmitting light through the sensor device, and detecting the presence of the analyte by detecting a pattern formed by diffraction of the transmitted light.

### Aims of the invention

The present invention aims to provide a method for assaying an analyte, which requires no labelling and which is very sensitive.

It is a further aim of the invention to provide a method, which is user-friendly and has a low manufacturing cost price. It is further aim of the invention to propose an improved method for assaying biomolecules.

### Summary of the invention

The present invention is directed to a method for analysing and determining an analyte within a sample comprising the steps of:
- providing a substrate, said substrate comprising an electrically conductive region and an analyte-specific recognition layer comprising receptor molecules capable of specifically interacting with the analyte; said conductive region having at least a first surface and a second surface,
wherein said first surface is operatively associated with the analyte-specific recognition layer,
- subjecting said substrate to said analyte, such that a specific interaction occurs between said analyte and said receptor molecules;
- directing electromagnetic radiation through said substrate; the method being characterized by further comprising the steps of
- measuring the intensity of said radiation absorbed or transmitted by said substrate as a function of the wavelength/frequency; and
- determining the presence of the analyte from a resulting change in the wavelength/frequency versus intensity of the radiation transmitted through or absorbed by the substrate in contact with the sample.

Said method can be used for affinity immunosensing and biosensing in general by optically monitoring of the recognition layer deposition, antibody immunization and the recognition of the antigen.

In an embodiment of this invention said conductive region consists essentially of at least one particle. Said particle is preferably smaller than the wavelength of the impinging radiation. The interaction between the analyte and the conductive regions affects the dielectric constant of the conductive region and the recognition layer, resulting in a change in the absorption or transmittance spectrum. Moreover, the interaction results in a change in the resonance frequency of the particle plasmon, since this is mainly determined by the dielectric function of the conductive region and the surrounding medium such as the recognition layer and the particle shape.

Preferably, the diameter of the particle is below 300 nm, below 200 nm, below 100 nm or below 50 nm.

In a further embodiment said interaction between said analyte and said recognition layers results in a change of the dielectric constant of said recognition layer.

In a further embodiment said substrate further comprises a support layer, and said second surface is operatively associated with said support layer. Said support layer is transparent or semi-transparent. In a further embodiment said conductive region comprises a metal. Said metal can be a metal inducing a plasmon effect. Said metal can be selected from the group consisting gold, silver and copper.

In a further embodiment said recognition layer comprises a self-assembling monolayer.

In a further embodiment said substrate has multiple conductive regions and said conductive regions are ordered in an array. Said substrate can be a microtitre plate. Said substrate can be used for high-throughput screening.

In a further embodiment the method further comprises the steps of
- providing a second substrate, said second substrate comprising a conductive region having at least a first surface and a second surface, and a recognition layer, wherein said first surface is operatively associated with said recognition layer,
- subjecting said second substrate to a preference sample,
- directing radiation through said conductive region and recognition layer of said second substrate,
- measuring the intensity of said radiation absorbed or transmitted by said second substrate as a function of the wavelength,
- comparing the intensity of said radiation absorbed or transmitted by said second substrate with the intensity of said radiation absorbed or transmitted by said first substrate in order to determine the presence of an analyte.

### Short description of the drawings

Figure 1 : experimental set-up as used in an embodiment of the method of the present invention.

Figure 2 : schematic representation of the method as described in conventional ELISA experiments.

Figure 3 : schematic representation of slides and the quartz cells as described in the preferred embodiment of the present invention.

Figure 4 : (a) absorbance spectra of Human Serum Albumin directly adsorbed on the thin gold film.
(b) difference spectra Human Serum Albumin directly adsorbed on the thin gold film and a thin gold on quartz.

Figure 5 : (a) Absorbance spectra of self-Assembled Monolayers of thiols on gold followed by adsorption of HSA at different concentrations and different times.
(b) difference spectra of the spectra of figure 5.

Figure 6 : Difference spectra used in immunosensing applications.

### Detailed description of the invention

In relation to the appended drawings the present invention is described in detail in the sequel. It is apparent however that a person skilled in the art can imagine several other equivalent embodiments within the scope of the present invention as defined by the claims.

The present invention discloses an improved method for detecting an analyte. The present invention may be used for sensing devices which have a higher sensitivity and which can be used to detect very low concentration of analyte.

In particular, this method can be used for affinity immunosensing and biosensing in general by optical monitoring of the linking layer deposition, recognition molecule immobilization and recognition of the analyte. This method is versatile, allowing applications in the liquid, gas phase, and allows quantitative in situ measurements.

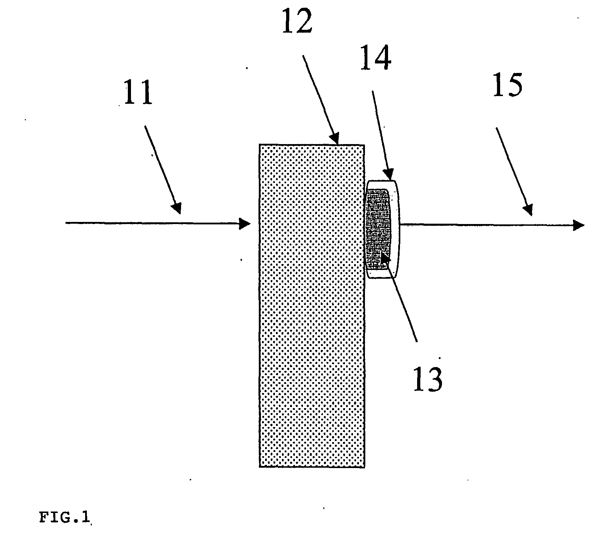

The present invention discloses a method for assaying an analyte in a sample (see figure 1), wherein said sample is brought into contact with a substrate (12) comprising an electrically conductive region (13) and a recognition layer (14), electromagnetic radiation (11) is directed through said substrate, and the intensity of said radiation (15) absorbed or transmitted by said substrate is measured as a function of wavelength/frequency. The presence of the analyte is determined by the resulting change in the spectrum (wavelength/frequency vs intensity) of the light transmitted through the substrate or absorbed by the substrate in contact with sample.

For the purpose of this invention, it should be understood that the words "absorbed radiation (or absorbance)" and "transmitted radiation (or transmittance)" can replace each other. The relation between the absorbance (A) and the transmittance (T) is given by : A = - log T

The present invention is directed to a method for analysing and determining an analyte within a sample comprising the steps of:
- providing a substrate, said substrate comprising an electrically conductive region and an analyte-specific recognition layer comprising receptor molecules capable of specifically interacting with the analyte; said conductive region having at least a first surface and a second surface,
wherein said first surface is operatively associated with the analyte-specific recognition layer,
- subjecting said substrate to said analyte, such that a specific interaction occurs between said analyte and said receptor molecules;
- directing electromagnetic radiation through said substrate; the method being characterized by further comprising the steps of
- measuring the intensity of said radiation absorbed or transmitted by said substrate as a function of the wavelength/frequency; and
- determining the presence of the analyte from a resulting change in the wavelength/frequency versus intensity of the radiation transmitted through or absorbed by the substrate in contact with the sample.

Said substrate can further comprise a support layer, said second surface of said conductive region can be operatively associated with said support layer.
The present invention can be used for assaying for an analyte in a sample.

Said analyte can be any kind of chemical molecule such as biomolecules, ions, cells. Said biomolecules can be hormones, proteins such as antibodies, antigens, steroids, nucleic acids, drug metabolites or microorganismes. Said sample can also be a "blank" sample, this means a sample without analyte or no sample.

The source of radiation can be any radiation source such as a lamp, a light emitting diode (LED) or a laser. In an embodiment, said radiation source should be able to provide a substantial amount of radiation in the wavelength range of the maximum absorbance (or minimum transmittance) wavelength of the substrate. Preferably, said radiation source provides radiation with a wavelength between 200 nm and 1500 nm. Preferably, said radiation source can generate radiation with a wavelength between 200 nm and 1000 nm. A LED, like a red LED or a blue LED can be used. Also other sources of radiation can be used.

Said radiation source can be a radiation source with a focussed beam (such as e.g. a laser) or can be a light source providing light with a broader spectrum. Preferably, said radiation source provides collimated radiation. Said radiation can be monochromatic.

Between said source of radiation and said substrate, several components can be present, such as lenses, slits, gratings.
The radiation source can be part of a commercially available UV-VIS spectrometer (or absorptiometer or colorimeter).

Said substrate comprises a conductive region and a recognition layer.

Said substrate can further comprise a support layer. Said support layer is for achieving at least mechanical support. Said support layer could be transparent or semi-transparent for the wavelength provided by the radiation source. Said support layer should have an optical transparency between 5 % and 95 %, preferably between 20% and 80 %, preferably, at least 80%. Said substrate can be made of, but is not limited hereto, glass, quartz, polymeric material (such as polycarbonate, polysulphonate, polymethyl-methacrylate). Preferably, said support layer is made of glass or quartz. Said support layer can be flat. Said support layer can also be part of a glass or quartz tube, a polymeric tube or a microtitre plate. The substrate can be integrated in a flow system. The substrate can also be a microtitre plate being part of a high-throughput screening system or ELISA tests.

Said conductive region comprises a first surface and a second surface. The first surface is operatively associated with said recognition layer. The second surface can be in contact with an external medium (such as e.g. air, a gas). In a preferred embodiment, the second surface is operatively associated with the support layer.

The conductive region comprises a conductive material. Preferably, said conductive material is a metal. Said metal can be, but is not limited hereto, gold, silver, copper. Any metal inducing a plasmon effect can be used of this invention. Other possible materials are conductive glass, conductive polymers or metallic nanoparticles.

Said conductive region can be a conductive layer or can be at least one particle. A conductive layer can have a thickness below 60 nm, below 50 nm, below 40 nm, below 30 nm below 20 nm or below 10 nm and preferably below 5 nm. Said layer can be continuous or can be discontinuous such that islands of conductive material are formed. A continuous layer can be uneven or even.

More preferably, said conductive region consists essentially of particles, more particularly micro- or nanoparticles. The size of the particles is lower than the wavelength of the radiation that impinges on the particle. The diameter of the particles is lower than 500 nm, lower than 400nm, preferably lower than 300 nm. Said thickness can also be lower than 200nm, lower than 100 nm, lower than 80 nm, lower then 50 nm, lower than 40 nm, lower than 30 nm or lower than 20 nm. The particle size can be determined by the deposition process. The shape of the particles can be spherical, but other structural and spatial configurations are not excluded. For instance, the particles can be slivers, cubic, ellipsoids, tubes and the like. The particles can be hollow. The particle can consist essentially of conductive material. The particle can also consist of a polymeric material covered with conductive material.

Said conductive region can be adapted in such a way that it can be optically tuneable. Optically tuneable layer means that the region has been produced in such a way that it has a predetermined thickness or a predetermined particle size, which corresponds to a preset value of the wavelength where the intensity of the absorbed radiation is sufficiently high. The desired thickness of the conductive region can be controlled by evaporation, sputtering, electroless plating or electroplating the conductive material.

In an embodiment, the second surface of the conductive region is operatively associated with a support layer. In an embodiment, the second surface of the conductive region can be deposited directly on the support layer. In another embodiment, at least one adhesion layer can be present between the support layer and the second surface of the conductive region. Said adhesion layer can improve the stability of the conductive region. Said adhesion layer can be, but is not limited hereto, a layer of self-assembling molecules such as, but not limited hereto, silane-based molecules or thiol-based molecules. Said adhesion layer can also comprise a layer of organic linker molecules (e.g. glue,...). Said adhesion layer can but does not have to have an effect on the absorption/transmittance characteristics of the first layer. Preferably, said adhesion layer is a non-metallic layer. Said substrate further comprises said recognition layer. Said recognition layer is operatively associated with the first surface of the conductive region. Said recognition layer is a layer comprising at least recognition molecules, also called receptive molecules. Said recognition molecules comprise one part of a specific binding pair and include anti-gen/antibody, enzyme/substrate, metal/chelator, bacteria/receptor, virus/receptor, hormone/receptor, oligonucleotide/RNA, DNA/RNA, RNA/RNA, oligonucleotide/DNA. The receptor molecules should be capable of specifically interacting with the analyte. This interaction can result in a change of the dielectric constant of the conductive region and the recognition layer. The recognition layer can also be a layer of cells being deposited directly on the first layer or on an intermediate layer. For the purpose of this application, intermediate layer should be understood as a layer being formed on the first surface of the conductive region. In many cases, the recognition layer is designed such that non-specific adsorption is essentially avoided.

Said recognition molecules can be deposited directly on the first surface of the conductive layer. Said recognition layer can also comprise a self-assembled monolayer (SAM) on which the recognition molecules can be bound (covalent or physical adsorption). Said Self-Assembled Monolayer can comprise at least two functional groups, a first group being selected such that it is operatively associated with the first surface of the conductive region and a second group being selected such that it interacts with the analyte.

An interaction between the recognition molecule and the analyte can, but does not necessarily have to, result in a change of the absorption spectrum of the substrate. When the substrate is subjected to the sample, the substrate is subjected to the radiation source such that the incident light impinges on the substrate, in particular on the conductive region and the recognition layer. The transmittance or the absorbance is determined. This can be done at a predetermined wavelength (for example the wavelength where the intensity is highest). Instead of measuring a change in peak in the spectrum, one could also determine a change in the integrated surface under the peak or measuring the shift in the spectrum. The measured transmittance or absorbance gives an indication of the presence of an analyte in the sample. E.g. when an analyte is present, the absorbance can increase or can decrease, the spectrum can shift, depending on the specific layers, on the interaction of the different layers, and on the analyte.

The transmittance or absorbance can be measured by a conventional absorptiometer (also called colorimeter) or spectrometer.

The measurement of absorbance or transmittance is advantageous as compared to fluorescence measurements since no labelling of the molecule is required. Consequently, the experimental procedure as described in this invention is simplified.

The use of a conventional light source such as a LED and the use of conventional absorptiometer result in method with a low manufacturing cost price. The invention can be performed in a solution e.g. water based, such that a flow system can be used. Otherwise, the experiments can be performed as "dry measurements".

Particularly in case of nano-particles, the absorption spectrum of metal nanoparticles is determined by both bulk interband absorption and particle plasmon resonances. The latter are collective oscillations of the conduction electrons on the surface of the small particle. The resonance frequency of a particle plasmon is determined mainly by the dielectric functions of the metal and the surrounding medium, respectively, and by the particle shape, i.e. the ratio of the principal axes. Resonances lead to narrow spectrally selective absorption and an enhancement of the local light field confined on and close to the surface of the metal particle. The surrounding medium influences the plasmon frequency and the amplitude of the absorption. The second mechanism playing a role in light absorption by small noble metal particles is photon interband absorption. It involves the promotion of an electron from the occupied d-level state in the noble metal to an empty state above the Fermi level. The absorption is strongly determined by the joint density of d and s states of the conduction electrons. Strong absorption indicates a "parallel" energy dispersion function. The different peaks n the spectrum can be assigned to different interband absorption peaks. Due to the large skin-depth of a few micron, the nanoparticles absorb light in the whole bulk area of the particle.

By increasing the dielectric constant near the nanoparticle surface an increase of the density for the electromagnetic field at the particle position enlarges the transition probability and as such the absorption for bulk transitions. This effect is only visible when the particle is smaller than the wavelength of the impinging light because such an object has too small a lateral extent to support any purely internal optical modes. The electric field operator internal to such a sphere is determined by the extended modes hence the dielectric constant of the surroundings. As the dielectric constant increases, an increased absorption is expected as experimentally verified. This may be different for particle plasmons because the dielectric constant of the surroundings also has a strong influence on the wavenumber and strength of the collective and evanescent mode of excitations. By coating the particles with a different material both a shift in frequency and absorption probability is seen.

Compared to SPR measurements, the method as described in this application can have the following advantages:
1) This method is simpler in set-up and can be made at a manufacturing low cost price. Moreover, this invention allows the different set-ups and can easily be integrated in different biological tools.
2) For the measurements, a normal UV-vis spectrometer can be used.
3) The intensity of the incoming light does not have to be focussed. A laser as incoming light is possible but is not necessary in our method.

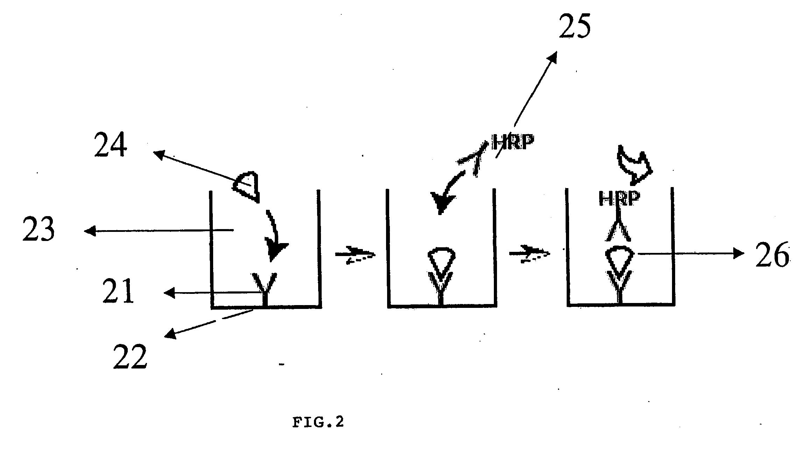

The method as described in the present invention has several advantages as compared to the conventional ELISA experiment, wherein an antibody (21) is immobilized on a microtiterplate (23) (see figure 2). This antibody cannot be detected by conventional UV-Vis measurements. The detection limit of UV-Vis measurements is not adequate to detect a thin layer or monolayer of proteins. In a next step the analyte or antigen (24) is recognized by the antibody. Also this event is not visible by UV-Vis measurements. Therefore a secondary antibody (25) with label (for example horseperoxidase) is used to couple to the other side of the antigen. Also this event is not visible. In a next step a substance is added which is converted by the label (HRP) to a colour in solution (26) which is or can give a quantitative estimation for the amount of antigen in the sample. This sequence of steps results in dilution curves, optimisations, calculations, time and money.

In the method as described in the invention, a thin layer of gold or nanoparticles is deposited on the bottom of an microtiter plate. The thin gold layer could be considered as being gold nanoparticles. Gold particles are deposited on the bottom of the micotitre plate. In a next step the antibody is coupled to the gold layer. The absorbance of the thin gold or the gold nanoparticles will be measured. This results in an absorption spectrum. In a next step the antibody is subjected to an external medium containing an antigen to be detected. The antigen will interact with the antibody resulting in a change in the absorption spectrum. The change can be an increase in intensity or a shift of the spectrum to lower/higher wavelengths. Consequently, the method as described in the present invention makes the assay faster, simpler, cheaper and more reliable.

### Description of a preferred embodiment of the invention

Ultrathin gold films were prepared via evaporation or via gold plating on a mercaptosilanized glass or quartz.

The glass or quartz substrates were cleaned by dissolving them in 2 M NaOH for 2 hours followed by a 7 minutes treatment with a 1/1/5 mixture of respectively H₂O₂ (30 %), NH₄OH (25 %) and ultrapure H₂O at 80 to 90 °C in order to achieve a freshly prepared and uniform oxide layer.

The compound 3-Mercaptopropylmethyltrimethoxysilane was dissolved in a 95:5 (v/v) solvent mixture at 2 %. The Self-Assembled mercaptosilane adhesion layers were formed by immersing the substrates in this solution for up to 72 h. Following immersion, the substrates were removed from the solution and rinsed with methanol, blow-dried with N₂ and heated for 10 min at 110°C. The coated substrates were stored in N₂ until gold evaporation or plating.

For the preparation of the gold films, two techniques can be used : (i) the *gold evaporation* was performed at a speed < 5 Å/sec with an Alcatel scm601. The final thickness on the mercaptosilanized substrates varied between 2 and 15 nm (average thickness),
(ii) the *electroless gold plating* was performed as described by Jin et al. (Jin, Y.; Kang, X.; Song, Y.; Zhang, B.; Cheng, G.; Dong, S. Anal. Chem. 2001, 73 (13), 2843). The mercaptosilanized substrates were overnight immersed in the colloidal gold solution mentioned above. The substrates having a monolayer of nanosized gold particles were consequently immersed in an aqueous 0.4 mM hydroxylamine hydrochloride and 0.1% HAuCl₄·3H₂O. All glassware was cleaned with 2 M NaOH for 2 hours. The substrates changed color from pink to purple to blue depending on the plating time therefore film thickness. After plating, the substrates were rinsed thoroughly with water, dried under a nitrogen stream, and were ready for measurements.

Self-Assembled Monolayers (SAMs) of 16-mercapto-1-hexadecanoic acid (16-MHA), 1-octadecanethiol (HS-C18) and 1-dodecanethiol (HS-C12) were realized by immersing the clean ultrathin gold substrates in a 1 mM thiol/ethanol solution for various times. The slides were consequently rinsed successively with ethanol and dried under a stream of nitrogen.

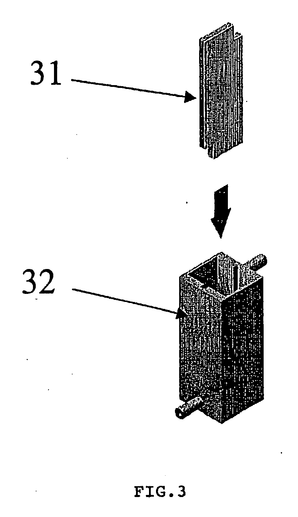

UV/VIS spectroscopic studies were carried out using a Shimatzu UV-1601PC with a slit width of 2 nm and data interval of 0.5 nm. The ultrathin gold-coated substrates were measured in air by placing the slides (31) perpendicular to the light beam. Characterization of this solution was performed in the quartz cells (32) schematically drawn in Figure 3.

AFM surface images were acquired in tapping mode under ambient conditions (PicoSPM, Molecular Imaging, USA). Si cantilevers having a spring constant between 1.2 and 5.5 N/m were used at resonance frequencies between 60 and 90 kHz.

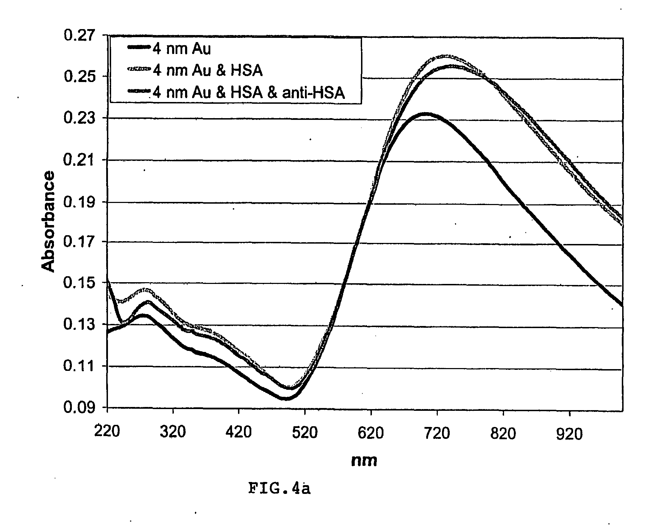

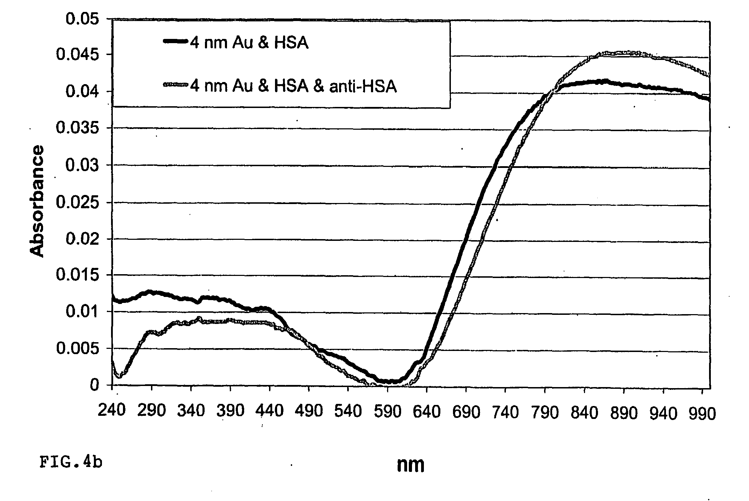

In a first experiment Human Serum Albumin was directly adsorbed on the thin gold film. In Figure 4a the raw absorbance spectra are shown. These measurements on evaporated thin gold on quartz were taken in ambient. The difference spectra are shown in Figure 4b. These spectra are background-corrected with the background being the absorbance spectra of the thin gold film. The deposition of HSA on 4 nm of evaporated gold was performed by a drop of 1.244 mg/mL in PB for 120 min followed by thoroughly rinsing with water and drying under a stream of N₂. The next step was the deposition of a drop of anti-HSA 250 µg/mL in PB for 180 min with the same rinsing and drying procedure. The absorbance changes and shifts and after each biosensing step. The increase in peak is a measure for the concentration of anti-HSA.

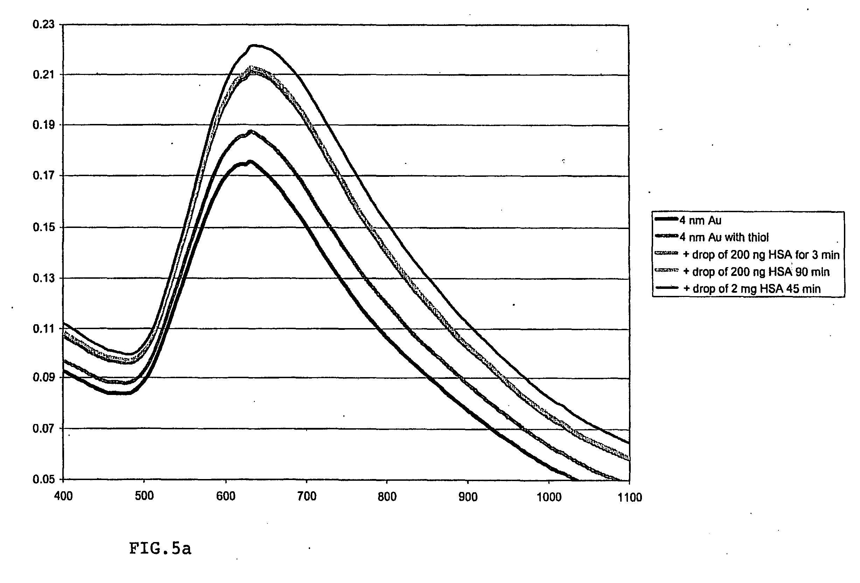

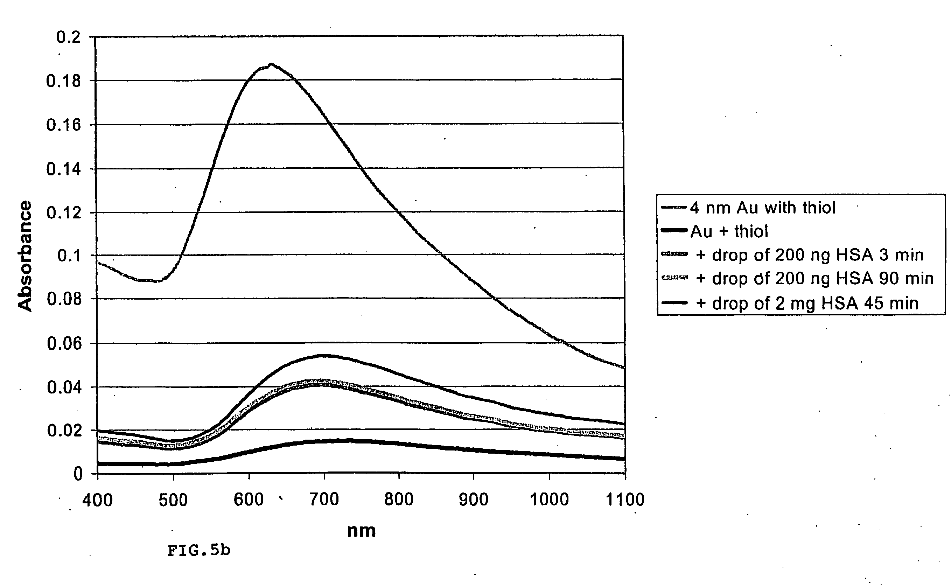

Self-Assembeld Monolayers of thiols were used to induce the adsorption or to covalently attach the bioreceptor molecules to the ultrathin gold. In a next experiment we used quartz with 4 nm of evaporated gold. The UV measurements were performed in air. The thin gold layer was immersed for 90 min in a 10 mM 1-dodecanethiol - ethanol solution. Sequentially the adsorption of HSA in function of time was followed. Different concentrations and different times were used. The adsorption was performed from a drop of the different concentrations of HSA in HBS. The absorbance peak shift is shown in Figure 5a. This shift is more pronounced in the difference spectra in Figure 5b. The dependence on the concentration is also clearly shown via the increase in the absorbance after introducing higher concentrations of HSA.

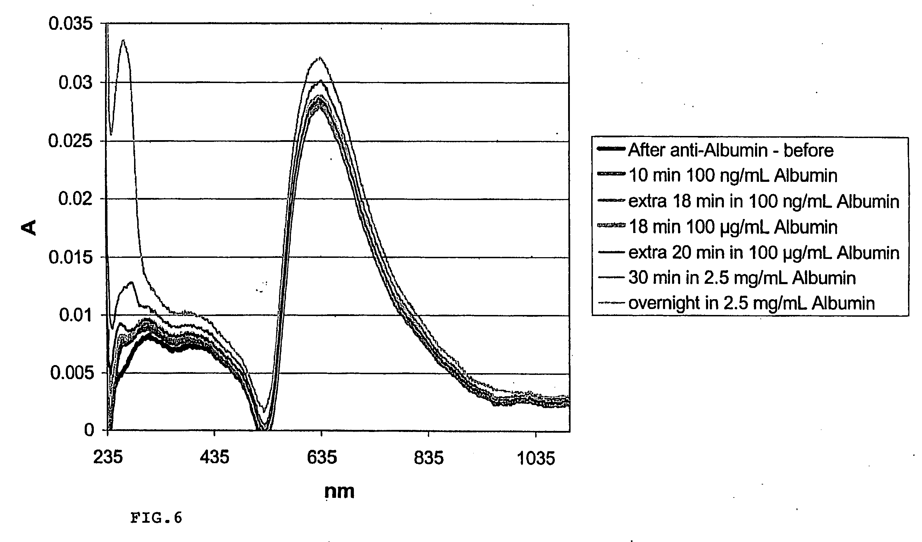

Immunosensing experiments were performed on a thin layer of plated gold (8 min of plating) and clearly show the potential of this sensing method for real biosensor applications (Figure 6 - difference spectra). A Self-Assembeld Monolayer of 16-MHA was formed on the thin gold film by a deposition of 25 minutes. The achieved carboxylic terminated SAM was activated via the EDC-NHS method with a mixture of 0.2 M/0.2 M EDC/NHS for 10 min. Consequently the amino groups of the lysine amino acids of anti-HSA (500 µg/mL in 10 mM acetate buffer pH = 5) were covalently coupled to the activated SAM surface. The not-reacted activated groups were blocked by rinsing for 7 min with 1 M ethanolamine and the not covalently bonded antibodies were removed by 2 min rinsing with 10 mM glycine. HCl buffer pH = 2.2. In this way a monolayer of anti-HSA on the surface can be observed. Again an enhancement around 270 nm is visible and a peak shift at 600 nm. These changes in the spectra can be used to determine the concentration of anti-HSA.

## Claims

1. A method for analysing and determining an analyte within a sample comprising the steps of:
- Providing a substrate, said substrate comprising an electrically conductive region and an analyte-specific recognition layer comprising receptor molecules capable of specifically interacting with the analyte,
said conductive region having at least a first surface and a second surface,
wherein said first surface is operatively associated with the analyte-specific recognition layer;
- Subjecting said substrate to said analyte, such that a specific interaction occurs between said analyte and said receptor molecules; and
- Directing electromagnetic radiation through said substrate;
the method being **characterized by** further comprising the steps of:
- Measuring the intensity of said radiation absorbed or transmitted by said substrate as a function of the wavelength/frequency; and
- determining the presence of the analyte from a resulting change in the wavelength/frequency versus intensity of the radiation transmitted through or absorbed by the substrate in contact with the sample.

2. A method as recited in claim 1 wherein light radiation is directed through said substrate.

3. A method as recited in any of the preceding claims wherein transmittance or absorbance is measured at a predetermined wavelength.

4. A method as recited in any of the preceding claims wherein the conductive region consists essentially of at least one particle.

5. A method as recited in claim 4 wherein the particle is smaller than the wavelength of the impinging radiation.

6. A method as recited in claim 4 or 5 wherein the particle has a diameter below 300 nm.

7. A method as recited in any of the preceding claims 4 to 6 wherein the particle has the shape of spheres, slivers, cubes, ellipsoids or tubes.

8. A method as recited in any of the preceding claims 4 to 7 wherein the particle consists essentially of conductive material or consists of a polymeric material covered with conductive material.

9. A method as recited in claim 8 wherein the particle consists essentially of conductive material selected from the group consisting of metal, conductive glass, conductive polymers and metallic nanoparticles.

10. A method as recited in any of the preceding claims wherein said specific interaction between said analyte and said receptor molecules results in a change of the dielectric constant of said recognition layer.

11. A method as recited in any of claims 1 to 10 wherein said substrate further comprises a support layer, and wherein said second surface of said substrate is operatively associated with said support layer.

12. A method as recited in claim 11 wherein said support layer is transparent or semi-transparent.

13. A method as recited in any of claims 1 to 12 wherein said conductive region comprises a metal.

14. A method as recited in claim 13 wherein the conductive region comprises at least one material selected from the group consisting of gold, silver or copper.

15. A method as recited in any of claims 1 to 14 wherein said recognition layer comprises a linker layer and a recognition molecule.

16. A method as recited in any of claims 1 to 15 wherein said recognition layer comprises a self-assembling monolayer.

17. A method as recited in any of claims 1 to 16 wherein said substrate has multiple conductive regions and wherein said conductive regions are ordered in an array.

18. A method as recited in any of claims 1 to 17 wherein the substrate is a microtitre plate.

19. A method as recited in any of claims 1 to 18 further comprising the steps of
- Providing a second substrate, said second substrate comprising a conductive region and a recognition layer as recited in claims 1 to 18,
- Subjecting said second substrate to a reference sample,
- Directing electromagnetic radiation through said second substrate,
- Measuring the intensity of said radiation absorbed or transmitted by said second substrate as a function of the wavelength, and then
- Comparing the intensity of said radiation absorbed or transmitted by said second substrate with the intensity of said radiation absorbed or transmitted by said first substrate in order to determine the presence of an analyte.

20. A method as recited in claim 19, wherein light radiation is directed through said substrate.

21. A method as recited in claim 19 or 20 wherein transmittance or absorbance is measured at a predetermined wavelength.

## Patentansprüche

1. Verfahren zum Analysieren und Bestimmen eines Analyten innerhalb einer Probe, welches die folgenden Verfahrensschritte umfasst:
- ein Bereitstellen eines Substrats, wobei das Substrat einen elektrisch leitfähigen Bereich und eine analyt-spezifische Erkennungslage aufweist, welche Rezeptormoleküle enthält, welche mit dem Analyten in eine spezifische Wechselwirkung zu treten in der Lage sind,
wobei der leitfähige Bereich mindestens eine erste Oberfläche und eine zweite Oberfläche aufweist,
wobei die erste Oberfläche operativ mit der analytspezifischen Erkennungslage assoziiert ist;
- das Substrat wird dem Analyten derart unterzogen, dass eine spezifische Wechselwirkung zwischen dem Analyten und den Rezeptormolekülen eintritt; und
- ein Hindurchschicken einer elektromagnetischer Strahlung durch das Substrat;
wobei das Verfahren **dadurch gekennzeichnet ist, dass** es weiterhin die folgenden Schritte aufweist:
- ein Messen der Intensität der von dem Substrat absorbierten oder durchgelassenen Strahlung als eine Funktion der Wellenlänge / Frequenz; und
- ein Bestimmen des Vorhandenseins des Analyten aus einer sich ergebenden Veränderung bei der Wellenlänge / Frequenz gegenüber der Intensität der Strahlung, die von dem Substrat durchgelassenen oder absorbierten worden ist, im Kontakt mit der Probe.

2. Verfahren gemäß Anspruch 1, bei welchem die Lichtstrahlung durch das besagte Substrat hindurch gelenkt wird.

3. Verfahren gemäß irgendeinem der vorhergehenden Ansprüche, bei welchem die Durchlässigkeit oder die Absorption bei einer vorherbestimmten Wellenlänge gemessen wird.

4. Verfahren gemäß irgendeinem der vorhergehenden Ansprüche, bei welchem der leitfähige Bereich im Wesentlichen aus mindestens einem Partikel besteht.

5. Verfahren gemäß Anspruch 4, bei welchem das Partikel kleiner ist als die Wellenlänge der auftreffenden Strahlung.

6. Verfahren gemäß Anspruch 4 oder 5, bei welchem das Partikel einen Durchmesser unterhalb von 300 nm aufweist.

7. Verfahren gemäß irgendeinem der vorhergehenden Ansprüche 4 bis 6, bei welchem das Partikel die Form von Kugeln, Splittern, Würfeln, Ellipsoiden oder von Rohren aufweist.

8. Verfahren gemäß irgendeinem der vorhergehenden Ansprüche 4 bis 7, bei welchem das Partikel im Wesentlichen aus einem leitfähigen Material besteht oder von einem polymeren Material gebildet wird, welches mit einem leitfähigen Material überzogen ist.

9. Verfahren gemäß Anspruch 8, bei welchem das Partikel im Wesentlichen aus einem leitfähigen Material besteht, welches ausgewählt wird aus der Gruppe bestehend aus einem Metall, leitfähigem Glas, leitfähigen Polymeren und metallischen Nanopartikeln.

10. Verfahren gemäß irgendeinem der vorhergehenden Ansprüche, bei welchem die spezifische Wechselwirkung zwischen dem Analyten und den Rezeptormolekülen zu einer Veränderung der dielektrischen Konstante der Erkennungslage führt.

11. Verfahren gemäß irgendeinem der Ansprüche 1 bis 10, bei welchem das Substrat weiterhin eine Trägerlage umfasst und bei welchem die zweite Oberfläche des Substrats operativ mit der Trägerlage assoziiert ist.

12. Verfahren gemäß Anspruch 11, bei welchem die Trägerlage durchsichtig oder halbdurchsichtig ist.

13. Verfahren gemäß irgendeinem der Ansprüche 1 bis 12, bei welchem der leitfähige Bereich ein Metall enthält.

14. Verfahren gemäß Anspruch 13, bei welchem der leitfähige Bereich mindestens ein Material enthält, welches ausgewählt wird aus der Gruppe bestehend aus Gold, Silber oder Kupfer.

15. Verfahren gemäß irgendeinem der Ansprüche 1 bis 14, bei welchem die Erkennungslage eine Verbindungslage und ein Erkennungsmolekül enthält.

16. Verfahren gemäß irgendeinem der Ansprüche 1 bis 15, bei welchem die Erkennungslage eine sich selbst anordnende Monolage enthält.

17. Verfahren gemäß irgendeinem der Ansprüche 1 bis 16, bei welchem das Substrat mehrfache leitfähige Bereiche aufweist und bei welchem die leitfähigen Bereiche in einer Gruppe angeordnet sind.

18. Verfahren gemäß irgendeinem der Ansprüche 1 bis 17, bei welchem das Substrat aus einer Mikrotitrierplatte besteht.

19. Verfahren gemäß irgendeinem der Ansprüche 1 bis 18, welches weiterhin die folgenden Verfahrensschritte umfasst:
- ein Bereitstellen eines zweiten Substrats, wobei das zweite Substrat einen elektrisch leitfähigen Bereich und eine Erkennungslage gemäß den Ansprüchen 1 bis 18 enthält,
- das zweite Substrat einer Referenzprobe unterziehen,
- ein Hindurchschicken einer elektromagnetischen Strahlung durch das zweite Substrat,
- ein Messen der Intensität der von dem zweiten Substrat absorbierten oder durchgelassenen Strahlung als eine Funktion der Wellenlänge, und alsdann
- ein Vergleichen der Intensität der von dem zweiten Substrat absorbierten oder durchgelassenen Strahlung mit der Intensität der von dem ersten Substrat absorbierten oder durchgelassenen Strahlung, um das Vorhandensein eines Analyten zu bestimmen.

20. Verfahren gemäß Anspruch 19, bei welchem die Lichtstrahlung durch das Substrat hindurch gelenkt wird.

21. Verfahren gemäß Anspruch 19 oder 20, bei welchem die Durchlässigkeit oder die Absorption bei einer vorherbestimmten Wellenlänge gemessen wird.

## Revendications

1. Procédé pour l'analyse et la détermination d'un analyte dans un échantillon comprenant les étapes consistant:
- à fournir un substrat, ledit substrat comprenant une région électriquement conductrice et une couche de reconnaissance spécifique à l'analyte comprenant des molécules réceptrices capables d'interagir spécifiquement avec l'analyte,
ladite région conductrice possédant au moins une première surface et une deuxième surface,
dans lequel ladite première surface est associée de manière active avec la couche de reconnaissance spécifique à l'analyte;
- à soumettre ledit substrat audit analyte, de sorte qu'une interaction spécifique se produit entre ledit analyte et lesdites molécules réceptrices; et
- à diriger un rayonnement électromagnétique à travers ledit substrat;
le procédé étant **caractérisé par** le fait de comprendre en outre les étapes consistant:
- à mesurer l'intensité dudit rayonnement absorbé ou transmis par ledit substrat en fonction de la longueur d'onde/fréquence; et
- à déterminer la présence de l'analyte à partir d'un changement résultant dans la longueur d'onde/fréquence par rapport à l'intensité du rayonnement transmis à travers ou absorbé par le substrat en contact avec l'échantillon.

2. Procédé suivant la revendication 1, dans lequel un rayonnement de lumière est dirigé à travers ledit substrat.

3. Procédé suivant l'une quelconque des revendications précédentes, dans lequel la transmittance ou l'absorbance est mesurée à une longueur d'onde prédéterminée.

4. Procédé suivant l'une quelconque des revendications précédentes, dans lequel la région conductrice est constituée essentiellement d'au moins une particule.

5. Procédé suivant la revendication 4, dans lequel la particule est plus petite que la longueur d'onde du rayonnement incident.

6. Procédé suivant la revendication 4 ou 5, dans lequel la particule possède un diamètre en dessous de 300 nm.

7. Procédé suivant l'une quelconque des revendications précédentes 4 à 6, dans lequel la particule a la forme de sphères, de rubans, de cubes, d'ellipsoïdes ou de tubes.

8. Procédé suivant l'une quelconque des revendications précédentes 4 à 7, dans lequel la particule est constituée essentiellement d'un matériau conducteur ou est constituée d'un matériau polymère couvert avec un matériau conducteur.

9. Procédé suivant la revendication 8, dans lequel la particule est constituée essentiellement d'un matériau conducteur choisi dans le groupe constitué de métal, de verre conducteur, de polymères conducteurs et de nanoparticules métalliques.

10. Procédé suivant l'une quelconque des revendications précédentes, dans lequel ladite interaction spécifique entre ledit analyte et lesdites molécules réceptrices conduit à un changement de la constante diélectrique de ladite couche de reconnaissance.

11. Procédé suivant l'une quelconque des revendications 1 à 10, dans lequel ledit substrat comprend en outre une couche support et dans lequel ladite deuxième surface dudit substrat est associée de manière active avec ladite couche support.

12. Procédé suivant la revendication 11, dans lequel ladite couche support est transparente ou semi-transparente.

13. Procédé suivant l'une quelconque des revendications 1 à 12, dans lequel ladite région conductrice comprend un métal.

14. Procédé suivant la revendication 13, dans lequel la région conductrice comprend au moins un matériau choisi dans le groupe constitué d'or, d'argent ou de cuivre.

15. Procédé suivant l'une quelconque des revendications 1 à 14, dans lequel ladite couche de reconnaissance comprend une couche de liaison et une molécule de reconnaissance.

16. Procédé suivant l'une quelconque des revendications 1 à 15, dans lequel ladite couche de reconnaissance comprend une monocouche auto-assembleuse.

17. Procédé suivant l'une quelconque des revendications 1 à 16, dans lequel ledit substrat possède de multiples régions conductrices et dans lequel lesdites régions conductrices sont ordonnées en un réseau.

18. Procédé suivant l'une quelconque des revendications 1 à 17, dans lequel le substrat est une plaque de microtitration.

19. Procédé suivant l'une quelconque des revendications 1 à 18, comprenant en outre les étapes consistant:
- à fournir un deuxième substrat, ledit deuxième substrat comprenant une région conductrice et une couche de reconnaissance suivant les revendications 1 à 18,
- à soumettre ledit deuxième substrat à un échantillon de référence,
- à diriger un rayonnement électromagnétique à travers ledit deuxième substrat,
- à mesurer l'intensité dudit rayonnement absorbé ou transmis par ledit deuxième substrat en fonction de la longueur d'onde, et ensuite
- à comparer l'intensité dudit rayonnement absorbé ou transmis par ledit deuxième substrat avec l'intensité dudit rayonnement absorbé ou transmis par ledit premier substrat dans le but de déterminer la présence d'un analyte.

20. Procédé suivant la revendication 19, dans lequel un rayonnement de lumière est dirigé à travers ledit substrat.

21. Procédé suivant la revendication 19 ou 20, dans lequel la transmittance ou l'absorbance est mesurée à une longueur d'onde prédéterminée.
